# EUROPEAN PATENT SPECIFICATION

(11) **EP 1 334 131 B1**
(45) Date of publication and mention of the grant of the patent: **16.07.2008**
(21) Application number: 01988737.1
(22) Date of filing: 22.10.2001
(51) Int. Cl.: C07K 16/00, C07K 1/00, C12P 21/08, A61K 39/40, A61K 39/395, A61K 39/00, A61K 39/02, C07K 16/12, C07K 14/31

(54) **MONOCLONAL ANTIBODIES TO THE MAP PROTEIN AND METHOD OF USE IN TREATING OR PREVENTING INFECTIONS**
MONOKLONALE ANTIKÖRPER GEGEN DAS MAP-PROTEIN UND VERFAHREN ZU DESSEN ANWENDUNG BEI DER BEHANDLUNG BZW. PRÄVENTION VON INFEKTIONEN
ANTICORPS MONOCLONAUX DIRIGES CONTRE LA PROTEINE MAP ET PROCEDE D'UTILISATION DE CEUX-CI DANS LE TRAITEMENT OU LA PREVENTION DES INFECTIONS

(30) Priority: 20.10.2000 US 241832 P; 21.03.2001 US 277287 P
(43) Date of publication of application: 13.08.2003
(73) Proprietor: Inhibitex, Inc., Alpharetta,GA 30004 (US)
(72) Inventor: PATTI, Joseph, M., Cumming, GA 30040 (US); DOMANSKI, Paul, Atlanta, GA 30319 (US); PATEL, Pratisksha, Suwanee, GA 30319 (US)
(74) Representative: Cripps, Joanna Elizabeth
(86) International application number: PCT/US2001/032550
(87) International publication number: WO 2002/034788

(56) References cited:
- WO-A-00/12131
- WO-A-02/077010
- WO-A1-96/39518
- US-A- 5 648 240
- US-A- 6 118 044
- DATABASE EMBL [Online] 14 November 1993 (1993-11-14), "Mus musculus (BALB/c) Ig mRNA clones 251-6." XP002328933 retrieved from EBI accession no. EM_PRO:MMRNA251 Database accession no. MMRNA251
- DATABASE EMBL [Online] 16 May 1996 (1996-05-16), "M.musculus mRNA for rearranged Ig heavy chain V region (J588 family; 129MD.43c)" XP002328934 retrieved from EBI accession no. EM_PRO:MMMD43C Database accession no. MMMD43C
- DATABASE EMBL [Online] 16 July 1999 (1999-07-16), "Staphylococcus aureus map-7 gene" XP002328935 retrieved from EBI accession no. EM_PRO:SAU243790 Database accession no. SAU243790
- DATABASE EMBL [Online] 11 February 1999 (1999-02-11), "Staphylococcus aureus mapN gene" XP002328936 retrieved from EBI accession no. EM_PRO:SAU132841 Database accession no. SAU132841
- VISAI L ET AL: "Monoclonal antibodies to CNA, a collagen-binding microbial surface component recognizing adhesive matrix molecules, detach Staphylococcus aureus from a collagen substrate" JOURNAL OF BIOLOGICAL CHEMISTRY, AMERICAN SOCIETY OF BIOLOGICAL CHEMISTS, BALTIMORE, MD, US, vol. 275, no. 51, 22 December 2000 (2000-12-22), pages 39837-39845, XP002307140 ISSN: 0021-9258
- PATTI J M ET AL: "CRITICAL RESIDUES IN THE LIGAND-BINDING SITE OF THE STAPHYLOCOCCUS AUREUS COLLAGEN-BINDING ADHESIN (MSCRAMM)" JOURNAL OF BIOLOGICAL CHEMISTRY, AMERICAN SOCIETY OF BIOLOGICAL CHEMISTS, BALTIMORE, MD, US, vol. 270, no. 20, 19 May 1995 (1995-05-19), pages 12005-12011, XP002044191 ISSN: 0021-9258
- NILSSON I-M ET AL: "Vaccination with recombinant fragment of collagen adhesin provides protection against Staphylococcus Aureus-mediated septic death" JOURNAL OF CLINICAL INVESTIGATION, NEW YORK, NY, US, vol. 101, no. 12, June 1998 (1998-06), pages 2640-2649, XP002183745 ISSN: 0021-9738
- KOFLER ET AL.: 'Mechanism of allergic cross-reactions-III. cDNA cloning and variable-region sequence analysis of two IgE antibodies specific for trinitrophenyl' MOL. IMMUNOL. vol. 29, no. 2, February 1992, pages 161 - 166, XP002908413
- MCGAVIN ET AL.: 'Identification of a staphylococcus aureus extracellular matrix-binding protein with broad specificity' INFECT. IMMUN. vol. 61, no. 6, June 1993, pages 2479 - 2485, XP001033794

### SEQUENCE LISTING

<110> INHIBITEX, INC.
<120> MONOCLONAL ANTIBODIES TO THE MAP PROTEIN AND METHOD OF USE IN TREATING AND PREVENTING INFECTIONS
<130> P06922WO00/BAS
<150> 60/277,287
   <151> 2001-03-21
<150> 60/241,832
   <151> 2000-10-20
<160> 6
<170> PatentIn version 3.0
<210> 1
   <211> 396
   <212> DNA
   <213> Staphylococcus aureus
<400> 1
   atgagaggat cgcatcacca tcaccatcac ggatcccaga ttccatatac aatcactgtg      60
   aatggtacaa gccaaaacat tttatcaagc ttaacattta ataagaatca acaaattagt     120
   tataaagata tagagaataa agttaaatca gttttatact ttaatagagg tattagtgat     180
   atcgatttaa gactttctaa gcaagcaaaa tacacggttc attttaagaa tggaacaaaa     240
   agagttgtcg atttgaaagc aggcattcac acagccgact taatcaatac aagtgacatt     300
   aaagcaatta gtgttaacgt agatactaaa aagcaagtga aagataaaga ggcaaaagca     360
   aatgttgtcg acctgcagcc aagcttaatt agctga                               396
<210> 2
   <211> 131
   <212> PRT
   <213> Staphylococcus aureus
<400> 2
   Met Arg Gly Ser His His His His His His Gly Ser Gln Ile Pro Tyr
   1               5                   10                  15
   Thr Ile Thr Val Asn Gly Thr Ser Gln Asn Ile Leu Ser Ser Leu Thr
               20                  25                  30
   Phe Asn Lys Asn Gln Gln Ile Ser Tyr Lys Asp Ile Glu Asn Lys Val
           35                  40                  45
   Lys Ser Val Leu Tyr Phe Asn Arg Gly Ile Ser Asp Ile Asp Leu Arg
       50                  55                  60
   Leu Ser Lys Gln Ala Lys Tyr Thr Val His Phe Lys Asn Gly Thr Lys
   65                  70                  75                  80
   Arg Val Val Asp Leu Lys Ala Gly Ile His Thr Ala Asp Leu Ile Asn
                   85                  90                  95
   Thr Ser Asp Ile Lys Ala Ile Ser Val Asn Val Asp Thr Lys Lys Gln
               100                 105                 110
   Val Lys Asp Lys Glu Ala Lys Ala Asn Val Val Asp Leu Gln Pro Ser
           115                 120                 125
   Leu Ile Ser
       130
<210> 3
   <211> 336
   <212> DNA
   <213> Staphylococcus aureus
<400> 3
   gatgttttga tgacccaaac tccactctcc ctgcctgtca gtcttggaga tcaagcctcc      60
   atctcttgca gatctagtca gaatattgct tttagtgatg gaaccaccta tttagaatgg     120
   tacctgcaga aaccaggcca gtctccaaag ctcctgatct acaaagtttc caaccgattt     180
   tctggggtcc cagacaggtt cagtggcagt ggatcaggga cagatttcac actcaagatc     240
   agcagtgtgg aggctgagga tctgggagtt tattactgct ttcaaggttc acatgttccg     300
   ctcacgttcg gtgctgggac caagctggag ttgaat                               336
<210> 4
   <211> 112
   <212> PRT
   <213> Staphylococcus aureus
<400> 4
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Asn Ile Ala Phe Ser
               20                  25                  30
   Asp Gly Thr Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Ser Val Glu Ala Glu Asp Leu Gly Val Tyr Tyr Cys Phe Gln Gly
                   85                  90                  95
   Ser His Val Pro Leu Thr Phe Gly Ala Gly Thr Lys Leu Glu Leu Asn
               100                 105                 110
<210> 5
   <211> 351
   <212> DNA
   <213> Staphylococcus aureus
<400> 5
   caggtccaac tgcagcagcc tggggctgag ctggtgaggc ctgggacttc agtgaaactg      60
   tcctgcaagg cttctggcta cacgttcacc agctactgga tgagctggac taagcagagg     120
   cctgaccaag gccttgagtg gattggaaga attgatcctt acgatagtga aactcactac     180
   aatcaaaagt tcaaggacaa ggtcatattg actgtggaca aatcctccag cacagcctac     240
   atgcaactca gcagcctgac atctgaggac tctgcggtct attactgtgc aagattggtc     300
   ccccactcca cggactactg gggccaaggc accactctca cagtctcctc a              351
<210> 6
   <211> 117
   <212> PRT
   <213> Staphylococcus aureus
<400> 6
   Gln Val Gln Leu Gln Gln Pro Gly Ala Glu Leu Val Arg Pro Gly Thr
   1               5                   10                  15
   Ser Val Lys Leu Ser Cys Lys Ala Ser Gly Tyr Thr Phe Thr Ser Tyr
               20                  25                  30
   Trp Met Ser Trp Thr Lys Gln Arg Pro Asp Gln Gly Leu Glu Trp Ile
           35                  40                  45
   Gly Arg Ile Asp Pro Tyr Asp Ser Glu Thr His Tyr Asn Gln Lys Phe
       50                  55                  60
   Lys Asp Lys Val Ile Leu Thr Val Asp Lys Ser Ser Ser Thr Ala Tyr
   65                  70                  75                      80
   Met Gln Leu Ser Ser Leu Thr Ser Glu Asp Ser Ala Val Tyr Tyr Cys
                   85                  90                      95
   Ala Arg Leu Val Pro His Ser Thr Asp Tyr Trp Gly Gln Gly Thr Thr
               100                 105                 110
   Leu Thr Val Ser Ser
           115

## Description

### Field of the Invention

The present invention relates in general to antibodies that have been generated against the MAP protein, a surface localized protein expressed by virtually every strain of *Staphylococcus aureus,* and in particular to monoclonal antibodies against Map10 protein and their use in the treatment of and protection against S. *aureus* infections.

### Background of the Invention

*Staphylococcus aureus* is a bacterial pathogen that is capable of colonizing a wide range of host tissues and causing a spectrum of infections that range from cutaneous lesions such as wound infections, impetigo, and furuncles to life-threatening conditions that include pneumonia, septic arthritis, sepsis, endocarditis, and biomaterial related infections. The successful colonization of the host is a process required for most microorganisms, including S. *aureus,* to cause infections in animals and humans. Microbial adhesion is the first crucial step in a series of events that can eventually lead to disease. Pathogenic microorganisms colonize the host by attaching to host tissues or serum conditioned implanted biomaterials, such as catheters, artificial joints, and vascular grafts, through specific adhesins present on the surface of the bacteria. MSCRAMM^{™}s (Microbial Surface Components Recognizing Adhesive Matrix Molecules) are a family of cell surface adhesins that recognize and specifically bind to distinct components in the host's extracellular matrix. Once the bacteria have successfully adhered and colonized host tissues, their physiology is dramatically altered and damaging components such as toxins and proteolytic enzymes are secreted. Moreover, adherent bacteria often produce a biofilm and quickly become more resistant to the killing effect of most antibiotics.

*S. aureus* is thus known to express a repertoire of different MSCRAMM^{™}s that can act individually or in concert to facilitate microbial adhesion to specific host tissue components. One such protein in known as the MAP protein, a surface localized protein expressed by virtually every S. *aureus* strain, as described for example in McGavin et al, Infect. Immun. p 2479-2485 (1993). However, it has still remained a problem to identify and utilize the information concerning MSCRAMM^{™}s from *S. aureus* such as the MAP protein because of the variability in the binding properties of the different MSCRAMM^{™}s and their role in infectivity and spread of bacterial infections. It has thus remained a highly desirable goal in the field of infectious diseases to develop compositions which are successful not only in preventing a wide variety of staph infections, but in facilitating a rapid or increased clearance of staph organisms from an infected host.

### Summary of the Invention

Accordingly, it is an object of the present invention to provide antibodies to the *S*. *aureus* MAP 10 protein to protect against staphylococcal infections.

It is also an object of the present invention to provide antibodies to the binding subdomains of the *S. aureus* MAP protein, including the Map10 protein, to protect against staphylococcal infections.

It is also an object of the present invention to provide a monoclonal antibody to the Map10 protein which is useful in preventing adherence of Staphylococcal bacteria and in facilitating a rapid clearance of such microbial organisms from an infected host through opsonophagocytic killing.

It is a further object of the present invention to provide antibodies and antisera which can recognize the MAP protein and thus which can be useful in methods of identifying and diagnosing staphylococcal infections.

It is a further object of the invention to provide amino acid sequences and the nucleic acid sequences which code for the variable light sequence and the variable heavy sequences of the monoclonal antibodies of the present invention.

These and other objects are provided by virtue of the present invention which comprises the isolation and use of monoclonal antibodies to the protein Map10, for the prevention and treatment of *Staphylococcus* infection. The discovery and isolation of anti-MAP antibodies in accordance with the present invention can thus be used in a double-edged attack against bacteria since these antibodies first prevent microbial adherence, and second facilitate a rapid clearance of the infectious organisms from the host through opsonophagocytic killing. Suitable compositions and vaccines based on the isolated MAP protein and antibodies raised thereto, as well as methods for their use, are also contemplated by the present invention.

These embodiments and other alternatives and modifications within the spirit and scope of the disclosed invention will become readily apparent to those skilled in the art from reading the present specification and/or the references cited herein, all of which are incorporated by reference.

### BRIEF DESCRIPTION OF THE DRAWING FIGURES

Figure 1 is a graphic representation of survival data of mice injected with monoclonal antibodies in accordance with the present invention.

Figure 2 is a graphic representation of survival data of mice injected with monoclonal antibodies in accordance with the present invention.

Figure 3 is a graphic representation of survival data of mice injected with monoclonal antibodies in accordance with the present invention.

Figure 4 is a graphic representation of survival data of mice injected with monoclonal antibodies in accordance with the present invention.

### DETAILED DESCRIPTION OF THE PREFERRED EMBODIMENTS

In accordance with the present invention, there is provided monoclonal antibodies which can bind to the MAP10 protein of *S. aureus* which have been isolated and purified by the present inventors, and which have been shown to protect against *S*. *aureus* infections. The MAP10 protein is a surface localized protein expressed by virtually every *S. aureus* strain. McGavin et al (McGavin et al, 1993, Infect. Immun. p 2479-2485) identified a 72 kDa surface protein, from *S. aureus* strain FDA 574, that binds a variety of host proteins including BSP, fibrinogen, fibronectin, vitronectin, and thrombospondin. The gene, designated *map,* was cloned and sequenced (U.S. Patent No. 5,648,240,-The *map* gene contains 6 repeated units, each subdomain (with roughly about 100-140 amino acids) displaying similarity to the peptide binding groove of MHC class II DRβ molecules from mammalian species. The MAP protein thus appears to have 6 separate subdomains each in the range of approximately 100-140 amino acids, and each which appear to contain a conserved amino acid sequence of about 35 residues. It was originally suggested that Map binding involved lectin-like activity (McGavin et al, 1993. Infect. Immun. p 2479-2485), however, subsequent studies demonstrated that the interaction between Map and its target proteins involves a protein-protein interaction (Jonsson et al. J. Biol. Chem. 1995, p.21457-21460). Using Southern blot and PCR techniques, a second *map* gene class was also discovered in *S. aureus* isolates. The proteins encoded by the second class of *map* genes displayed heterogeneity with respect to size as reflected by the presence or absence of the number of repeating units. In accordance with the present invention, the inventors have determined that the Map10 protein of S. *aureus* (see the sequence set forth below as SEQ ID NO:2) appears to be the minimal binding region of the whole MAP protein, and thus can be used to isolate and obtain monoclonal and other types of antibodies which can be useful in preventing binding of staphylococcal bacteria such as *S. aureus* to eukaryotic cells and thus be capable or treating or preventing *S. aureus-type* infections.

Accordingly, the present invention relates to an isolated and/or purified monoclonal antibody which can bind to the MAP10 protein and which thus can be useful in methods of preventing and treating staphylococcal infection when used in amounts effective to prevent or treat such infections. These monoclonal antibodies may be produced using, e.g., the method of Kohler and Milstein, Nature 256:495-497 (1975), or other suitable ways known in the field, and in addition can be prepared as chimeric, humanized, or human monoclonal antibodies in ways that would be well known in this field. Still further, monoclonal antibodies may be prepared from a single chain, such as the light or heavy chains, and in addition may be prepared from active fragments of an antibody which retain the binding characteristics of the whole antibody. By active fragments is meant an antibody fragment which has the same binding specificity as a complete antibody which binds to the MAP10 protein, and the term "antibody" as used herein is meant to include said fragments. Additionally, antisera prepared using monoclonal or polyclonal antibodies in accordance with the invention are also contemplated and may be prepared in a number of suitable ways as would be recognized by one skilled in the art.

As indicated above, antibodies to the MAP10 protein may be prepared in a number of suitable ways that would be well known in the art, such as the well-established Kohler and Milstein method described above which can be utilized to generate monoclonal antibodies to the MAR10 protein. In one such method, mice are injected intraperitoneally once a weeks for a prolonged period with a purified recombinant MAP10 protein, followed by a test of blood obtained from the immunized mice to determine reactivity to the purified MAP protein. Following identification of mice reactive to MAP10, lymphocytes isolated from mouse spleens are fused to mouse myeloma cells to produce hybridomas positive for the antibodies against MAP10 which are then isolated and cultured, following by purification and isotyping.

In order to generate monoclonal antibodies in accordance with the invention, it is preferred that these be generated using recombinantly prepared Map10 proteins using conventional methods well known in the art. For example, one such method employs the use of *E. coli* expression vector pQE-30 as an expression vector for cloning and expressing recombinant proteins and peptides. DNA preparation, purification, restriction digestion, agarose gel electrophoresis and ligation may be performed using standard methods, and the resulting recombinant MAP segments may be isolated and purified and then utilized to generate monoclonal antibodies in the manner described above.

In the preferred method, using PCR, the first subdomain of *map* can be amplified such as from *S. aureus* FDA 574 genomic DNA and subcloned into the *E. coli* expression vector PQE-30 (Qiagen), which allows for the expression of a recombinant fusion protein containing six histidine residues. This vector may be subsequently transformed into a suitable *E. coli* strain, grown in a fermentor to a suitable optical density (e.g., OD₆₀₀) and induced with a suitable compound such as 0.2 mM isopropyl-1-beta-D galactoside (IPTG). The cells may then be harvested using a hollow-fiber assembly (e.g., of pore size 0.45 µm) and the cell paste frozen prior to lysing using a suitable press (e.g., 2 passes through a French Press @ 1100psi). Lysed cells can then be spun down to remove cell debris, and isolating a suitable MAP protein, or a suitable subdomain such as Map10, using suitable methods such as chelating columns and appropriate washing and eluting. The MAP protein may also undergo an endotoxin removal protocol. Additional steps may be carried as needed to further purify the product, and antibodies generated to the purified MAP protein as described further below. One such Map10 protein isolated through this method has the sequence as set forth in SEQ ID NO:2, and is encoded by nucleic acids having the sequence as set forth in SEQ ID NO:1, or degenerates thereof.

In accordance with the invention, monoclonal antibodies to the MAP proteins can be produced by a number of suitable ways. In one preferred method, the purified Map10 subdomain was used generate a panel of murine monoclonal antibodies. In this preferred method, a group of Balb/C mice received a series of subcutaneous immunizations of 135 µg of Map10 in solution or mixed with adjuvant such as Subcutaneous Freund's Complete or Incomplete adjuvant. Three days after the final boost of adjuvant, the spleens were removed, teased into a single cell suspension and the lymphocytes harvested. The lymphocytes were then fused to a SP2/0-Ag14 myeloma cell line (ATCC #1581). Cell fusion, subsequent plating and feeding were performed according to the Production of Monoclonal Antibodies protocol from Current Protocols in immunology (Chapter 2, Unit 2.).

Any clones that were generated from the fusion were then screened for specific anti-Map. 10 antibody production using a standard ELISA assay. Positive clones were expanded and tested further, and clones were identified which produced single cell clones that generated anti-Map.10 antibodies.

Next, hybridoma cells were grown in RPMI/DMEM. 1X Nutridoma-SP media containing 2mM sodium pyruvate, 4mM L-glutamine and 2X penicillin-streptomycin to 2-3 liter culture volumes. Hybridoma supernatants were then harvested by centrifugation. The supernatants were filtered through 0.45 µM filters and the IgG was affinity purified using protein G chromatography. The monoclonal antibodies were eluted using 0.1M glycine, pH 2.7 and immediately neutralized with one tenth volume of 2M Tris, pH 8.0. The purified IgG was then dialyzed against 1X D-phosphate buffered saline, pH 7.4. If needed, the purified antibody was concentrated and aliquots frozen. Monoclonal antibodies produced in accordance with the invention include an antibody having a variable light sequence as indicated in SEQ ID NO:4, encoded by nucleic acids having the sequence of SEQ ID NO:3, or degenerates thereof, and a variable heavy sequence as indicated in SEQ ID NO:6, encoded by nucleic acids having the sequence of SEQ ID NO:5, or degenerates thereof.

In addition to monoclonal antibodies, the present invention also contemplates generating polyclonal antibodies from MAP¹⁰ Such polyclonal antibodies may be generated in any of a number of suitable ways well known in the art, such as the introduction of a purified Map10 protein peptide into a suitable animal host, followed by isolation and purification of the generated antibodies produced in the host animal.

Although production of antibodies using recombinant forms of the MAP 10 protein is preferred, antibodies may be generated from natural isolated and purified MAP proteins or peptides as well, and monoclonal or polyclonal antibodies can be generated using the natural MAP proteins in the same manner as described above to obtain such antibodies. Still other conventional ways are available to generate the MAP antibodies of the present invention using recombinant or natural purified MAP proteins, as would be recognized by one skilled in the art.

As would be recognized by one skilled in the art, the antibodies of the present invention may also be formed into suitable pharmaceutical compositions for administration to a human or animal patient in order to treat or prevent an infection caused by staphylococcal bacteria. Pharmaceutical compositions containing the antibodies of the present invention, or effective fragments thereof, may be formulated in combination with any suitable pharmaceutical vehicle, excipient or carrier that would commonly be used in this art, including such as saline, dextrose, water, glycerol, ethanol, other therapeutic compounds, and combinations thereof. As one skilled in this art would recognize, the particular vehicle, excipient or carrier used will vary depending on the patient and the patient's condition, and a variety of modes of administration would be suitable for the compositions of the invention, as would be recognized by one of ordinary skill in this art. Suitable methods of administration of any pharmaceutical composition disclosed in this application include, but are not limited to, topical, oral, anal, vaginal, intravenous, intraperitoneal, intramuscular, subcutaneous, intranasal and intradermal administration.

For topical administration, the composition is formulated in the form of an ointment, cream, gel, lotion, drops (such as eye drops and ear drops), or solution (such as mouthwash). Wound or surgical dressings, sutures and aerosols may be impregnated with the composition. The composition may contain conventional additives, such as preservatives, solvents to promote penetration, and emollients. Topical formulations may also contain conventional carriers such as cream or ointment bases, ethanol, or oleyl alcohol.

Additional forms of antibody compositions, and other information concerning compositions, methods and applications with regard to other MSCRAMM^{™}s will also apply to the present invention involving antibodies to the MAP MSCRAMM^{™} and are disclosed, for example, in U.S. Patent 6,288,214 (Hook et al.).

The antibody compositions of the present invention which are generated against the MAP protein or its subdomains such as Map10 may also be administered with a suitable adjuvant in an amount effective to enhance the immunogenic response against the conjugate. For example, suitable adjuvants may include alum (aluminum phosphate or aluminum hydroxide), which is used widely in humans, and other adjuvants such as saponin and its purified component Quil A, Freund's complete adjuvant, and other adjuvants used in research and veterinary applications. Still other chemically defined preparations such as muramyl dipeptide, monophosphoryl lipid A, phospholipid conjugates such as those described by Goodman-Snitkoff et al. J. lmmunol. 147:410-415 (1991) encapsulation of the conjugate within a proteoliposome as described by Miller et al., J. Exp. Med. 176:1739-1744 (1992) and encapsulation of the protein in lipid vesicles such as Novasome^{™} lipid vesicles (Micro Vescular Systems, Inc., Nashua, NH) may also be useful.

In any event, the antibody compositions of the present invention will thus be useful for interfering with, modulating, inhibiting binding interactions between staphylococcal bacteria and the MAP protein on host cells, or in displacing staphylococcal bacteria which has become bound to MAP on host cells. Accordingly, the present invention will have particular applicability in developing compositions and methods of preventing or treating staphylococcal infection, and in inhibiting binding of staphylococcal bacteria to eukaryotic cells.

In accordance with the present invention, methods are provided for preventing or treating a staphylococcal infection which comprise administering an effective amount of an antibody to the Map10 protein as described above in amounts effective to treat or prevent the infection. As also indicated above, the Map10 antibodies in accordance with the invention are doubly effective in that they have been observed not only to prevent bacterial adherence, but also to increase the opsonophagocytic activity which leads to increasing killing of the organism and rapid clearance of the infection from the host. Accordingly, administration of the antibodies of the present invention in any of the conventional ways described above (e.g., topical, parenteral, intramuscular, etc.), and will thus provide an extremely useful method of treating or preventing staphylococcal infections in human or animal patients. By effective amount is meant that level of antibody titer that will be sufficient to either prevent adherence of the bacteria, to inhibit binding of staph bacteria to host cells, or, in the case of a prior infection, that amount that will be sufficient to enhance opsonophagocytic killing and promote clearance of the bacteria from the host cells so as to treat the infection. As would be recognized by one of ordinary skill in this art, the level of antibody titer needed to be effective in treating or preventing staphylococcal infection will vary depending on the nature and condition of the patient, and/or the severity of the pre-existing staphylococcal infection.

In addition to the use of antibodies to the MAP protein in methods to treat or prevent *S. aureus* infection as described above, the present invention contemplates the use of these antibodies in a variety of ways, including the detection of the presence of S. *aureus* to diagnose a staph infection, whether in a patient or on medical equipment which may also become infected. In accordance with the invention, a preferred method of detecting the presence of staph infections involves the steps of obtaining a sample suspected of being infected by one or more staphylococcal bacteria species or strains, such as a sample taken from an individual, for example, from one's blood, saliva, tissues, bone, muscle, cartilage, or skin. The cells can then be lysed, and the DNA extracted, precipitated and amplified. Following isolation of the sample, diagnostic assays utilizing the Map10 antibodies of the present invention may be carried out to detect the present of *S. aureus,* and such assay techniques for determining such presence in a sample are well known to those skilled in the art and include methods such as radioimmunoasssay, Western blot analysis and ELISA assays. In general, in accordance with the invention, a method of diagnosing an *S. aureus* infection is contemplated wherein a sample suspected of being infected with *S. aureus* infection has added to it a MAP protein antibody in accordance with the present invention, and *S. aureus* is indicated by antibody binding to the MAP proteins in the sample.

Accordingly, antibodies in accordance with the invention may be used for the specific detection of staphylococcal map proteins, for the prevention of infection from staph bacteria, for the treatment of an ongoing infection, or for use as research tools. The term "antibodies" as used herein includes monoclonal, polyclonal, chimeric, single chain, bispecific, simianized, and humanized or primatized antibodies as well as Fab fragments, such as those fragments which maintain the binding specificity of the antibodies to the Map10 proteins, including the products of an Fab immunoglobulin expression library. Generation of any of these types of antibodies or antibody fragments is well known to those skilled in the art. In the present case, monoclonal antibodies to MAP proteins have been generated and isolated and shown to protect against staphylococcal infection.

Any of the above described antibodies may be labeled directly with a detectable label for identification and quantification of staph bacteria. Labels for use in immunoassays are generally known to those skilled in the art and include enzymes, radioisotopes, and fluorescent, luminescent and chromogenic substances, including colored particles such as colloidal gold or latex beads. Suitable immunoassays include enzyme-linked immunosorbent assays (ELISA).

Alternatively, the antibody may be labeled indirectly by reaction with labeled substances that have an affinity for immunoglobulin. The antibody may be conjugated with a second substance and detected with a labeled third substance having an affinity for the second substance conjugated to the antibody. For example, the antibody may be conjugated to biotin and the antibody-biotin conjugate detected using labeled avidin or streptavidin. Similarly, the antibody may be conjugated to a hapten and the antibody-hapten conjugate detected using labeled anti-hapten antibody. These and other methods of labeling antibodies and assay conjugates are well known to those skilled in the art.

Antibodies to the MAP protein may also be used in production facilities or laboratories to isolate additional quantities of the proteins, such as by affinity chromatography. For example, the antibodies of the invention may also be utilized to isolate additional amounts of the MAP protein.

The isolated antibodies of the present invention, or active fragments thereof, may also be utilized in the development of vaccines for passive immunization against staph infections. Further, when administered as pharmaceutical composition to a wound or used to coat medical devices or polymeric biomaterials *in vitro* and *in vivo,* the antibodies of the present invention, are useful in those cases where there is a previous staph infection because of the ability of this antibody to enhance opsonophagocytic killing of bacteria. In addition, the antibody may be modified as necessary so that, in certain instances, it is less immunogenic in the patient to whom it is administered. For example, if the patient is a human, the antibody may be "humanized" by transplanting the complimentarity determining regions of the hybridoma-derived antibody into a human monoclonal antibody as described, e.g., by Jones et al., Nature 321:522-525 (1986) or Tempest et al. Biotechnology 9:266-273 (1991).

Medical devices or polymeric biomaterials to be coated with the antibodies, proteins and active fragments described herein include, but are not limited to, staples, sutures, replacement heart valves, cardiac assist devices, hard and soft contact lenses, intraocular lens implants (anterior chamber or posterior chamber), other implants such as corneal inlays, kerato-prostheses, vascular stents, epikeratophalia devices, glaucoma shunts, retinal staples, scleral buckles, dental prostheses, thyroplastic devices, laryngoplastic devices, vascular grafts, soft and hard tissue prostheses including, but not limited to, pumps, electrical devices including stimulators and recorders, auditory prostheses, pacemakers, artificial larynx, dental implants, mammary implants, penile implants, cranio/facial tendons, artificial joints, tendons, ligaments, menisci, and disks, artificial bones, artificial organs including artificial pancreas, artificial hearts, artificial limbs, and heart valves; stents, wires, guide wires, intravenous and central venous catheters, laser and balloon angioplasty devices, vascular and heart devices (tubes, catheters, balloons), ventricular assists, blood dialysis components, blood oxygenators, urethral/ureteral/urinary devices (Foley catheters, stents, tubes and balloons), airway catheters (endotracheal and tracheostomy tubes and cuffs), enteral feeding tubes (including nasogastric, intragastric and jejunal tubes), wound drainage tubes, tubes used to drain the body cavities such as the pleural, peritoneal, cranial, and pericardial cavities, blood bags, test tubes, blood collection tubes, vacutainers, syringes, needles, pipettes, pipette tips, and blood tubing.

It will be understood by those skilled in the art that the term "coated" or "coating", as used herein, means to apply the antibody or active fragment, or pharmaceutical composition derived therefrom, to a surface of the device, preferably an outer surface that would be exposed to streptococcal bacterial infection. The surface of the device need not be entirely covered by the protein, antibody or active fragment.

In a preferred embodiment, the antibodies may also be used as a passive vaccine which will be useful in providing suitable antibodies to treat or prevent a staphylococcal infection. As would be recognized by one skilled in this art, a vaccine may be packaged for administration in a number of suitable ways, such as by parenteral (i.e., intramuscular, intradermal or subcutaneous) administration or nasopharyngeal (i.e., intranasal) administration. One such mode is where the vaccine is injected intramuscularly, e.g., into the deltoid muscle, however, the particular mode of administration will depend on the nature of the bacterial infection to be dealt with and the condition of the patient. The vaccine is preferably combined with a pharmaceutically acceptable carrier to facilitate administration, and the carrier is usually water or a buffered saline, with or without a preservative. The vaccine may be lyophilized for resuspension at the time of administration or in solution.

The preferred dose for administration of an antibody composition in accordance with the present invention is that amount will be effective in preventing of treating a staphylococcal infection, and one would readily recognize that this amount will vary greatly depending on the nature of the infection and the condition of a patient. As indicated above, an "effective amount" of antibody or pharmaceutical agent to be used in accordance with the invention is intended to mean a nontoxic but sufficient amount of the agent, such that the desired prophylactic or therapeutic effect is produced. As will be pointed out below, the exact amount of the antibody or a particular agent that is required will vary from subject to subject, depending on the species, age, and general condition of the subject, the severity of the condition being treated, the particular carrier or adjuvant being used and its mode of administration, and the like. Accordingly, the "effective amount" of any particular antibody composition will vary based on the particular circumstances, and an appropriate effective amount may be determined in each case of application by one of ordinary skill in the art using only routine experimentation. The dose should be adjusted to suit the individual to whom the composition is administered and will vary with age, weight and metabolism of the individual. The compositions may additionally contain stabilizers or pharmaceutically acceptable preservatives, such as thimerosal (ethyl(2-mercaptobenzoate-S)mercury sodium salt) (Sigma Chemical Company, St. Louis, MO).

When used with suitable labels or other appropriate detectable biomolecule or chemicals, the monoclonal antibodies described herein are useful for purposes such as *in vivo* and *in vitro* diagnosis of staphylococcal infections or detection of staphylococcal bacteria. Laboratory research may also be facilitated through use of such antibodies. Various types of labels and methods of conjugating the labels to the antibodies of the invention are well known to those skilled in the art, such as the ones set forth below.

For example, the antibody can be conjugated to a radiolabel such as, but not restricted to, ³²P, ³H, ¹⁴C, ³⁵S, ¹²⁵I, or ¹³¹I. Detection of a label can be by methods such as scintillation counting, gamma ray spectrometry or autoradiography. Bioluminescent labels, such as derivatives of firefly luciferin, are also useful. The bioluminescent substance is covalently bound to the protein by conventional methods, and the labeled protein is detected when an enzyme, such as luciferase, catalyzes a reaction with ATP causing the bioluminescent molecule to emit photons of light. Fluorogens may also be used to label proteins. Examples of fluorogens include fluorescein and derivatives, phycoerythrin, allo-phycocyanin, phycocyanin, rhodamine, and Texas Red. The fluorogens are generally detected by a fluorescence detector.

The location of a ligand in cells can be determined by labeling an antibody as described above and detecting the label in accordance with methods well known to those skilled in the art, such as immunofluorescence microscopy using procedures such as those described by Warren and Nelson (Mol. Cell. Biol., 7: 1326-1337, 1987).

As indicated above, the monoclonal antibodies of the present invention, or active portions or fragments thereof, are particularly useful for interfering with the initial physical interaction between a staphylococcal pathogen responsible for infection and a mammalian host, such as the adhesion of the bacteria to mammalian extracellular matrix proteins such as the MAP protein, and this interference with the physical interaction may be useful both in treating patients and in preventing or reducing bacteria infection on in-dwelling medical devices to make them safer for use.

In another embodiment of the present invention, a kit which may be useful in isolating and identifying staphylococcal bacteria and infection is provided which comprises the antibodies of the present invention in a suitable form, such as lyophilized in a single vessel which then becomes active by addition of an aqueous sample suspected of containing the staphylococcal bacteria. Such a kit will typically include a suitable container for housing the antibodies in a suitable form along with a suitable immunodetection reagent which will allow identification of complexes binding to the MAP antibodies of the invention. For example, the immunodetection reagent may comprise a suitable detectable signal or label, such as a biotin or enzyme that produces a detectable color, etc., which normally may be linked to the antibody or which can be utilized in other suitable ways so as to provide a detectable result when the antibody binds to the antigen.

In short, the antibodies of the present invention which bind to the MAP protein or active fragments thereof are thus extremely useful in treating or preventing staphylococcal infections in human and animal patients and in medical or other in-dwelling devices. Accordingly, the present invention relates to methods of identifying and isolating antibodies which can bind to the MAP protein and which can be used in methods of treatment of staph infections which involve opsonophagocytic killing of the bacteria. Antibodies which are identified and/or isolated using the present method, such as the MAP antibody which can bind the Map protein and which can prevent or treat a staph infection thus is part of the present invention

### EXAMPLES

The following examples are provided which exemplify aspects of the preferred embodiments of the present invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1. Isolation and Sequencing of MAP protein and DNA

Using PCR, the first subdomain of *map* was amplified from *S. aureus* FDA 574 genomic DNA and subcloned into the *E. coli* expression vector PQE-30 (Qiagen), which allows for the expression of a recombinant fusion protein containing six histidine residues. This vector was subsequently transformed into the *E. coli* strain ATCC 55151, grown in a 15-liter fermentor to an optical density (OD₆₀₀) of 0.7 and induced with 0.2 mM isopropyl-1-beta-D galactoside (IPTG) for 4 hours. The cells were harvested using an AG Technologies hollow-fiber assembly (pore size of 0.45 µm) and the cell paste frozen at -80° C. Cells were lysed in 1X PBS (10mL of buffer/1 g of cell paste) using 2 passes through the French Press @ 1100psi. Lysed cells were spun down at 17,000rpm for 30 minutes to remove cell debris. Supernatant was passed over a 5-mL HiTrap Chelating (Pharmacia) column charged with 0.1M NiCl₂. After loading, the column was washed with 5 column volumes of 10mM Tris, pH 8.0, 100mM NaCI (Buffer A). Protein was eluted using a 0-100% gradient of 10mM Tris, pH 8.0, 100mM NaCl, 200mM imidazole (Buffer B) over 30 column volumes. Map10 eluted at ~13% Buffer B (~26mM imidazole). Absorbance at 280nm was monitored. Fractions containing Map10 were dialyzed in 1x PBS.

The protein was then put through an endotoxin removal protocol. Buffers used during this protocol were made endotoxin free by passing over a 5-mL Mono-Q sepharose (Pharmacia) column. Protein was divided evenly between 4x 15mL tubes. The volume of each tube was brought to 9mL.with Buffer A. 1mL of 10% Triton X-114 was added to each tube and incubated with rotation for 1 hour at 4°C. Tubes were placed in a 37°C water bath to separate phases. Tubes were spun down at 2,000rpm for 10 minutes and the upper aqueous phase from each tube was collected and the detergent extraction repeated. Aqueous phases from the 2nd extraction were combined and passed over a 5-mL IDA chelating (Sigma) column, charged with 0.1M NiCl₂ to remove remaining detergent. The column was washed with 9 column volumes of Buffer A before the protein was eluted with 3 column volumes of Buffer B. The eluant was passed over a 5-mL Detoxigel (Sigma) column and the flow-through collected and reapplied to the column. The flow-through from the second pass was collected and dialyzed in 1x PBS. The purified product was analyzed for concentration, purity and endotoxin level before administering to mice.

### Example 2. Production and Isolation of the MAP Monoclonal Antibody

The purified Map10 protein was used generate a panel of murine monoclonal antibodies. Briefly, a group of Balb/C mice received a series of subcutaneous immunizations of 135 µg of Map.10 protein in solution or mixed with adjuvant as described below:

| Injection | Day | Amount (µg) | Route | Adjuvant |
|---|---|---|---|---|
| Primary | 0 | 135 | Subcutaneous | Freund's Complete |
| Boost #1 | 39 | 135 | Subcutaneous | Freund's Incomplete |
| Boost #2 | 63 | 135 | Subcutaneous | Freund's Incomplete |
| Final Boost | 130 | 135 | Subcutaneous | Freund's Incomplete |

Three days after the final boost, the spleens were removed, teased into a single cell suspension and the lymphocytes harvested. The lymphocytes were then fused to a SP2/0-Ag14 myeloma cell line (ATCC #1581). Cell fusion, subsequent plating and feeding were performed according to the Production of Monoclonal Antibodies protocol from Current Protocols in Immunology (Chapter 2, Unit 2.).

Any clones that were generated from the fusion were then screened for specific anti-Map.10 antibody production using a standard ELISA assay. Positive clones were expanded and tested further. Ten positive clones were originally identified, however four eventually died, leaving only six that were eventually single cell cloned by limiting dilution. Single cell clones were tested for activity and only four of the original six clones produced single cell clones that generated anti-Map.10 antibodies.

### Antibody Scale-up and Purification

Hybridoma cells were grown in RPMI/DMEM, 1X Nutridoma-SP media containing 2mM sodium pyruvate, 4mM L-glutamine and 2X penicillin-streptomycin to 2-3 liter culture volumes. Hybridoma supernatants were then harvested by centrifugation. The supernatants were filtered through 0.45 µM filters and the IgG was affinity purified using protein G chromatography. The monoclonal antibodies were eluted using 0.1M glycine, pH 2.7 and immediately neutralized with one tenth volume of 2M Tris, pH 8.0. The purified IgG was then dialyzed against 1X D-phosphate buffered saline, pH 7.4. If needed, the purified antibody was concentrated and aliquots frozen.

### Staphylococcus aureus strains

S. *aureus* cells were taken from a frozen glycerol stock and were inoculated onto a single blood agar plate and grown for 24 hours at 37°C. A single colony was then selected and inoculated onto a new blood agar plate. This was repeated for approximately 30 plates per 20 mls of final frozen stock. The plates were then incubated for 24 hours at 37°C. Following incubation, the colonies were scraped off the surface of each plate into a 50 ml tube containing 10 mls of 1XPBS, while gently vortexing to remove the bacteria from the scraper (20-30 plates per each 10 mls of PBS). An additional 10 mls of 1XPBS was then added to the 10 mls of bacterial suspension, while vigorously vortexing to facilitate separation of any agar debris from the bacteria. The suspension was pelleted by centrifugation, 3500xg at 4°C for 10 minutes. The bacteria was washed three times in D-PBS and resuspended to the desired volume of media. The bacterial stock was placed into 1 ml aliquots by snap freezing in an ethanol/dry bath and placed in -80°C freezer. CFU/ml concentration of frozen stock was determined by thawing 1 ml of stock, centrifuging 3500xg at 4°C for 10 minutes, decanting supernatant from tube and resuspending pellet in 1 ml of 1XPBS. Serial dilutions from 10⁻¹ to 10⁻⁸ using 100µl of prior dilution and 900µl of 1XPBS were made and 50µl of 10⁻⁴ to 10⁻⁸ dilutions were plated in duplicate on blood agar plates and incubated for 37°C for 16-18 hours. The absorbance at 600nm for 10⁻¹ to 10⁻² was measured and recorded. The CFU/ml was determined (CFU/ml=(average # colonies X dilution factor)/0.050 mls) and averaged for each dilution to determine the average CFU/ml. On the day of injection two 1ml aliquots were thawed, combined into one tube and vortexed. Following injections, 10⁻⁵, 10⁻⁶, and 10⁻⁷ dilutions of the sample preparation were plated on blood agar plates to determine the CFU/ml injected. The amount of bacteria injected was 2.2 X10⁸ CFU/ml S. *aureus* Barnett.

### Animal, Sex, Species, Number, Age and Source

Female Balb/C mice (5-6 weeks of age) were purchased from Taconic Quality Laboratory Animals and Services for Research (Germantown, NY). Animals were allowed to acclimate for at least 14 days prior to initiation of treatment. Upon arrival, the mice were examined, group housed (5 / cage) in polycarbonate shoe box cages with absorbent bedding. All mice were placed on a 12 hour light-dark cycle under the required husbandry standards found in the NIH Guide for the Care and Use of Laboratory Animals.

### Identification and Randomization

All animals were uniquely identified using tail tattoos prior to dosing. Prior to initiation of treatment, the animals were individually weighed and their health was evaluated. Mice were randomized and assigned to treatment groups using stratified body weights.

### MAP Specific Monoclonal Antibodies (Mab), Isotype:

H01 MAP.10 Mab, IgG₁
H04 MAP.10 Mab, IgG₁
H07 MAP.10 Mab, IgG₁
H10 MAP.10 Mab, IgG₁

### Control

The control was Phosphate Buffered Saline, pH 7.4 (PBS), purchased from Life Technologies, Inc. (Cat. No. 10010-023; Lot No. 1078749).

The experimental design for the experiments are shown below:

### Experimental Design

**Table 1.**

| | | TREATMENT | | | | | CHALLENGE | | |
|---|---|---|---|---|---|---|---|---|---|
| Group # | No. of ice | Antibody | Dose | Route | Frequency | Time Point | Bacteria | Stock Dilution. | Route |
| 1 | 10 | H01MAP.10 | 0.5 mg | IP | once | -18 hr. | Barnett | 2.2 x 10⁸ | IV |
| 2 | 15 | H04 MAP.10 | 0.5 mg | IP | once | -18 hr. | Barnett | 2.2 x 10⁸ | IV |
| 3 | 15 | H07 MAP.10 | 0.5 mg | IP | once | -18 hr. | Barnett | 2.2 x 10⁸ | IV |
| 4 | 15 | H10 MAP.10 | 0.5 mg | IP | once | -18 hr. | Barnett | 2.2 x 10⁸ | IV |
| 5 | 15 | D-PBS | N/A | IP | once | -18 hr. | Barnett | 2.2 x 10⁸ | IV |

### DATA

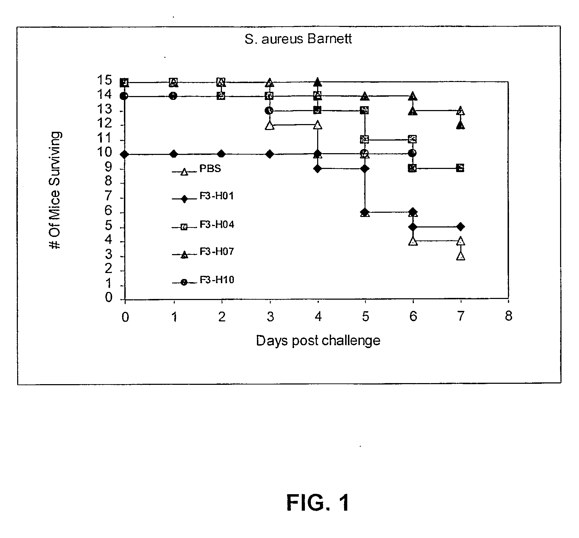

Mice were treated by intraperitoneal (IP; 0.5ml) injection with 0.5 mg of monoclonal H01, H04, H07, H10 or PBS. Eighteen hours after IgG administration, the mice were challenged with a single intravenous (IV) injection of *S. aureus* strain Barnett. The mice were then followed for 7 days (Fig. 1) and the survival data of each group of mice was analyzed by a logrank test known as Mantel-Cox. The data are summarized in the Table 2.

**Table 2.**

| **Experimental Group** | **Statistic** |
|---|---|
| H01 vs. PBS | p = 0.5837 |
| H04 vs. PBS | p = 0.0282 |
| H07 vs. PBS | p = 0.0005 |
| H10 vs. PBS | p = 0.0377 |

Monoclonal H07 exhibited the best protective effects in the mouse bacteremia model. Monoclonal H01 had no efficacy, whereas monoclonals H04 and H10 produced significant protection compared to control, but somewhat less effective when compared to H07. The data presented here clearly demonstrated that monoclonal antibodies against MAP protect against S. *aureus* infections.

### Example 3. Comparison of Mab H07 and Mab10 against several S. aureus strains

### Antibody Scale-up and Purification

Hybridoma cells were grown in RPMI/DMEM, 1X Nutridoma-SP media containing 2mM sodium pyruvate, 4mM L-glutamine and 2X penicillin-streptomycin to 2-3 liter culture volumes. Hybridoma supernatants were then harvested by centrifugation. The supernatants were filtered through 0.45 µM filters and the IgG was affinity purified using protein G chromatography. The monoclonal antibodies was eluted using 0.1M glycine, pH 2.7 and immediately neutralized with one tenth volume of 2M Tris, pH 8.0. The purified IgG was then dialyzed against 1X D-phosphate buffered saline, pH 7.4. If needed, the purified antibody was concentrated and aliquots frozen.

### Staphylococcus aureus

S. *aureus* cells were taken from a frozen glycerol stock and were inoculated onto a single blood agar plate and grown for 24 hours at 37°C. A single colony was then selected and inoculated onto a new blood agar plate. This was repeated for approximately 30 plates per 20 mls of final frozen stock. The plates were then incubated for 24 hours at 37°C. Following incubation, the colonies were scraped off the surface of each plate into a 50 ml tube containing 10 mls of 1XPBS, while gently vortexing to remove the bacteria from the scraper (20-30 plates per each 10 mls of PBS). An additional 10 mls of 1XPBS was then added to the 10 mls of bacterial suspension, while vigorously vortexing to facilitate separation of any agar debris from the bacteria. The suspension was pelleted by centrifugation, 3500xg at 4°C for 10 minutes. The bacteria was washed three times in D-PBS and resuspended to the desired volume of media. The bacterial stock was placed into 1 ml aliquots by snap freezing in an ethanol/dry bath and placed in -80°C freezer. CFU/ml concentration of frozen stock was determined by thawing 1 ml of stock, centrifuging 3500xg at 4°C for 10 minutes, decanting supernatant from tube and resuspending pellet in 1 ml of 1XPBS. Serial dilutions from 10⁻¹ to 10⁻⁸ using 100µl of prior dilution and 900µl of 1XPBS were made and 50µl of 10⁻⁴ to 10⁻⁸ dilutions were plated in duplicate on blood agar plates and incubated for 37°C for 16-18 hours. The absorbance at 600nm for 10⁻¹ to 10⁻² was measured and recorded. The CFU/ml was determined (CFU/ml=(average # colonies X dilution factor)/0.050 mls) and averaged for each dilution to determine the Average CFU/ml. On the day of injection two 1ml aliquots were thawed, combined into one tube and vortexed. Following injections, 10⁻⁵, 10⁻⁶, and 10⁻⁷ dilutions of the sample preparation were plated on blood agar plates to determine the CFU/ml injected. The amount of bacteria injected was 1.4 X10⁹ CFU/ml *S. aureus* Barnett, 2.3 X 10⁸ CFU/ml *S. aureus* ATCC 25923, 4.9 X 10⁷ CFU/ml *S. aureus* ATCC 49230.

### Animal, Sex, Species, Number, Age and Source

Female Balb/C mice (5-6 weeks of age) were purchased from Taconic Quality Laboratory Animals and Services for Research (Germantown, NY). Animals were allowed to acclimate for at least 14 days prior to initiation of treatment. Upon arrival, the mice were examined, group housed (5 / cage) in polycarbonate shoe box cages with absorbent bedding. All mice were placed on a 12 hour light-dark cycle under the required husbandry standards found in the NIH Guide for the Care and Use of Laboratory Animals.

### Identification and Randomization

All animals were uniquely identified using tail tattoos prior to dosing. Prior to initiation of treatment, the animals were individually weighed and their health was evaluated. Mice were randomized and assigned to treatment groups using stratified body weights.

### MAP Specific Mabs

H07 MAP.10 Mab
H10 MAP.10 Mab

### Control

The control was Phosphate Buffered Saline, pH 7.4 (PBS), purchased from Life Technologies, Inc. (Cat. No. 10010-023; Lot No. 1078749).

The experimental design for the experiments are shown below:

### Experimental Design

**Table 3**

| | | TREATMENT | | | | | CHALLENGE | | |
|---|---|---|---|---|---|---|---|---|---|
| Group # | No. of Mice | Antibody | Dose | Route | Frequency | Time Point | Bacteria | Ino-culum CFU/MI | Route |
| 1 | 15 | H07 MAP.10 | 0.5 mg | IP | once | -18 hr. | Bamett | 1.4X10⁹ | IV |
| 2 | 15 | H07 MAP.10 | 0.5 mg | IP | once | -18 hr. | ATCC 25923 | 2.3X10⁸ | IV |
| 3 | 15 | H07 MAP.10 | 0.5 mg | IP | once | -18 hr. | ATCC 49230 | 4.5X10⁷ | IV |
| 4 | 15 | H10 MAP.10 | 0.5 mg | IP | once | -18 hr. | Bamett | 1.4X10⁹ | IV |
| 5 | 15 | H10 MAP.10 | 0.5 mg | IP | once | -18 hr. | ATCC 25923 | 2.3X10⁸ | IV |
| 6 | 15 | H10 MAP.10 | 0.5 mg | IP | once | -18 hr. | ATCC 49230 | 4.5X10⁷ | IV |
| 7 | 15 | D-PBS | N/A | IP | once | -18 hr. | Bamett | 1.4X10⁹ | IV |
| 8 | 15 | D-PBS | N/A | IP | once | -18 hr. | ATCC 25923 | 2.3X10⁸ | IV |
| 9 | 15 | D-PBS | N/A | IP | once | -18 hr. | ATCC 49230 | 4.5X10⁷ | IV |

### DATA

Mice were treated by intraperitoneal (IP; 0.5ml) injection with 0.5 mg of monoclonal H07, H10 or PBS. Eighteen hours after IgG administration, the mice were challenged with a single intravenous (IV) injection of S. *aureus* strain Barnett. The mice were then followed for 7 days and the survival data of each group of mice was analyzed by a logrank test known as Mantel-Cox. The data are summarized in Table 4.

**Table 4.**

| **Test Agents** | **Challenge Bacterial Strain** | **Statistic** |
|---|---|---|
| H07 vs. PBS | ATCC 25923 | p < 0.0001 |
| H 10 vs. PBS | ATCC 25923 | p = 0.0078 |
| H07 vs. PBS | ATCC 49230 | p < 0.0008 |
| H10 vs. PBS | ATCC 49230 | p = 0.0062 |
| H07 vs. PBS | Barnett | p = 0.0173 |
| H10 vs. PBS | Barnett | p = 0.0396 |

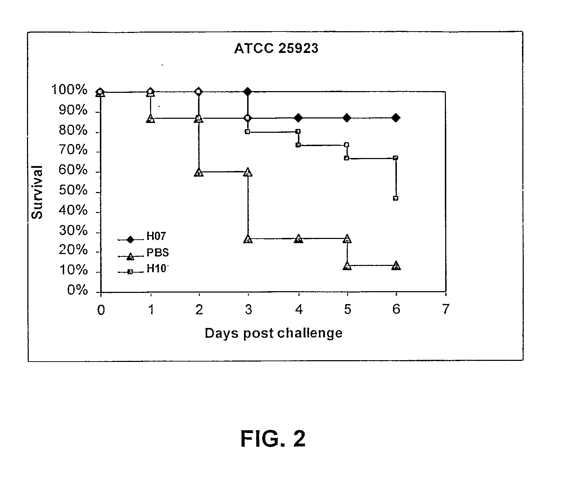

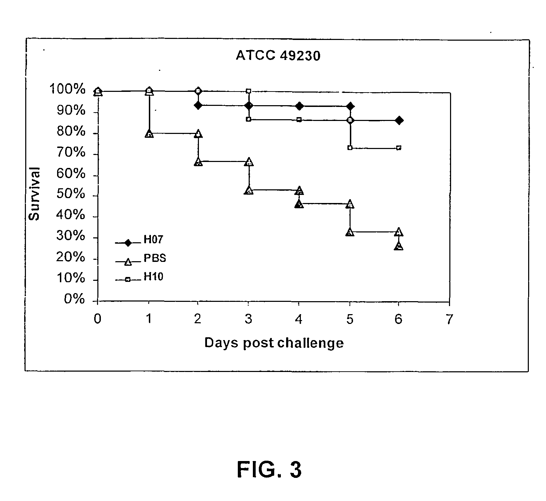

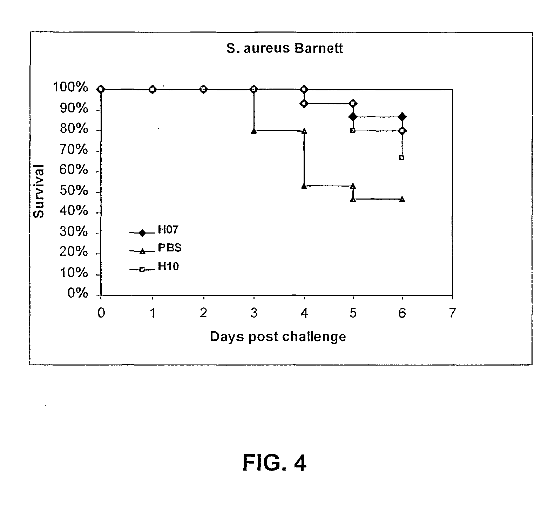

These data clearly demonstrate that a single infusion of Map.10 monoclonal antibody H07 and H10 can significantly prevent bacteremia mediated death against multiple strains of S. *aureus* in a relevant in vivo model. The data are shown in Figures 2, 3, and 4.

### Example 4. Isolation and Sequencing of Variable Region Sequences

Messenger RNA was isolated from Map H07 hybridoma cells using the Fast Track 2.0 kit (Invitrogen; Cat. No. K4500). Briefly, 1.4x10⁸ hybridoma cells cultured in DMEM-10 medium with 10 % FBS were washed with PBS, pelleted by centrifugation then lysed in detergent containing Protein/RNase Degrader. PolyA⁺ mRNA was isolated by affinity purification on oligo-dT cellulose. Synthesis of first strand cDNA was accomplished using 5µg of mRNA and reverse transcriptase in a cDNA synthesis kit (Novagen; Cat. No. 69001-3) containing 20 pmol of 3' oligonucleotide mouse-specific primers (Novagen; Cat. Nos. 69796 and 69812) for each variable heavy and variable light chain. A portion (5 to 50 ng) of the cDNA was amplified by the polymerase chain reaction (PCR) using the PCR Reagent System (Life Technologies; Cat. No. 10198-018) and a mouse variable heavy and light chain specific primer set (Novagen; Cat. No. 70081-3, 5 pmol each) for 30 cycles (94 C hot start then cycles of 94 C for 1 min, 50 C for 1 min and 72 C for 1min). PCR products were fractionated electrophoretically in a 1% ultra pure agarose gel in sodium acetate buffer and visualized by ethidium bromide staining. PCR fragments matching the predicted size were excised from the gel and purified using BIO 101 Geneclean spin columns (Cat. No. 1101-400) for ligation into the pCR2.1-TOPO (Invitrogen) plasmid, followed by transformation into competent TOP10 E. coli. (Invitrogen; Cat. No. K4500). After isolating plasmid DNA using QlAprep Spin Miniprep Kit (QIAGEN; Cat. No. 27106), positive clones with inserts were identified by restriction endonuclease digestion and agarose gel electrophoresis, followed by sequencing on an ABI automated sequencer using M13 Forward and M13 Reverse primers.

The results of the sequencing were as follows: Amino acids representing a CDR are underlined Amino acids representing a CDR are underlined

## Claims

1. An isolated antibody which binds to the Map10 protein from S. aureus, wherein the Map10 protein has a sequence according to SEQ ID NO:2.

2. An antibody according to Claim 1, wherein said antibody prevents S. aureus infection in a human or animal.

3. An antibody according to Claim 1, wherein said antibody inhibits binding of staphylococcal bacteria to eukaryotic cells.

4. An antibody according to Claim 1, wherein said antibody is suitable for parenteral, oral, intranasal, subcutaneous, aerosolized or intravenous administration in a human or animal.

5. An antibody according to Claim 1 wherein the antibody is a monoclonal antibody.

6. An antibody according to Claim 5 wherein the monoclonal antibody is of a type selected from the group consisting of chimeric, humanized and human monoclonal antibodies.

7. An antibody according to Claim 5 wherein the antibody is a single chain monoclonal antibody.

8. An antibody according to Claim 1 which comprises an antibody fragment having the same binding specificity of an antibody which binds to the S. aureus MAP protein.

9. An antibody according to Claim 1 having a variable light sequence according to SEQ ID NO:4.

10. An antibody according to Claim 1 having a variable light sequence encoded by a nucleic acid sequence according to SEQ ID NO: 3 or degenerates thereof.

11. An antibody according to Claim 1 having a variable heavy sequence according to SEQ ID NO: 6.

12. An antibody according to Claim 1 having a variable heavy sequence encoded by a nucleic acid sequence according to SEQ ID NO: 5 or degenerates thereof.

13. An antibody according to Claim 1 wherein the antibody is a polyclonal antibody.

14. Isolated antisera containing an antibody according to Claim 1.

15. A diagnostic kit comprising an antibody according to Claim 1 and means for detecting binding by that antibody.

16. A diagnostic kit according to Claim 15 wherein said means for detecting binding comprises a detectable label that is linked to said antibody.

17. An *in vitro* method of diagnosing an infection of S, aureus comprising adding an antibody according to Claim 1 to a sample suspected of being infected with S. aureus, and determining if antibodies have bound to the sample.

18. A pharmaceutical composition for treating or preventing an infection of S. aureus comprising an effective amount of the antibody of Claim 1 and a pharmaceutically acceptable vehicle, carrier or excipient.

19. Use of an antibody according to Claim 1 in the preparation of a medicament for treating or preventing an infection of *S.aureus* in a human or animal patient.

20. Use of an isolated S. aureus Map10 protein having an amino acid sequence according to SEQ ID NO:2, in the preparation of a medicament for treating or preventing an infection of *S.aureus,.*

21. An *in vitro* method of identifying antibodies to the Map10 protein comprising adding an isolated Map10 protein having an amino acid sequence according to SEQ ID NO:2 to a sample suspected of containing anti-MAP antibodies, and determining if antibodies have bound to the added Map10 protein.

22. An isolated antibody according to Claim 1 that has the ability to bind to an amino acid sequence coded by the nucleic acid sequence of SEQ ID NO :1 or degenerates thereof.

23. An isolated S. aureus Map10 protein having an amino acid sequence according to SEQ ID NO:2.

24. An isolated protein according to Claim 23 having an amino acid sequence encoded by a nucleic acid sequence according to SEQ ID NO :1 or degenerates thereof.

25. An isolated nucleic acid sequence consisting of a nucleic acid sequence according to SEQ ID NO:1

26. An antibody according to claim 1 for use in treating or preventing an infection of S. aureus in a human or animal patient.

27. An isolated S. aureus Map10 protein having an amino acid sequence according to SEQ ID NO:2 for treating or preventing an infection of *S*.*aureus*..

## Patentansprüche

1. Isolierter Antikörper, der an das Map10-Protein von S. aureus bindet, worin das Map10-Protein eine Sequenz gemäß Seq.-ID Nr. 2 aufweist.

2. Antikörper nach Anspruch 1, worin der Antikörper eine S.-aureus-Infektion in einem Menschen oder einem Tier verhindert.

3. Antikörper nach Anspruch 1, worin der Antikörper die Bindung von Staphylokokken-Bakterien an eukaryotische Zellen hemmt.

4. Antikörper nach Anspruch 1, worin der Antikörper zur parenteralen, oralen, intranasalen, subkutanen, Aerosol- oder intravenösen Verabreichung an einen Menschen oder ein Tier geeignet ist.

5. Antikörper nach Anspruch 1, worin der Antikörper ein monoklonaler Antikörper ist.

6. Antikörper nach Anspruch 5, worin der monoklonale Antikörper ein Typ ist, der aus der aus chimären, humanisierten und menschlichen monoklonalen Antikörpern bestehenden Gruppe ausgewählt ist.

7. Antikörper nach Anspruch 5, worin der Antikörper ein einkettiger monoklonaler Antikörper ist.

8. Antikörper nach Anspruch 1, der ein Antikörperfragment umfasst, das die gleiche Bindungsspezifität aufweist wie ein Antikörper, der an das S.-aureus-MAP-Protein bindet.

9. Antikörper nach Anspruch 1, der eine variable leichte Sequenz gemäß Seq.-ID Nr. 4 aufweist.

10. Antikörper nach Anspruch 1, der eine variable leichte Sequenz aufweist, für die eine Nucleinsäuresequenz gemäß Seq.-ID Nr. 3 oder eine degenerierte Form davon kodiert.

11. Antikörper nach Anspruch 1, der eine variable schwere Sequenz gemäß Seq.-ID Nr. 6 aufweist.

12. Antikörper nach Anspruch 1, der eine variable schwere Sequenz aufweist, für die eine Nucleinsäuresequenz gemäß Seq.-ID Nr. 5 oder eine degenerierte Form davon kodiert.

13. Antikörper nach Anspruch 1, worin der Antikörper ein polyklonaler Antikörper ist.

14. Isoliertes Antiserum, das einen Antikörper nach Anspruch 1 enthält.

15. Diagnoseset, das einen Antikörper nach Anspruch 1 und Mittel zur Detektion der Bindung durch diesen Antikörper umfasst.

16. Diagnoseset nach Anspruch 15, worin das Mittel zur Detektion der Bindung eine detektierbare Markierung umfasst, die an diesen Antikörper gebunden ist.

17. In-vitro-Verfahren zur Diagnose einer S.-aureus-Infektion, umfassend das Zusetzen eines Antikörpers nach Anspruch 1 zu einer Probe, die vermutlich mit S. aureus infiziert ist, und das Bestimmen, ob Antikörper an die Probe gebunden haben.

18. Pharmazeutische Zusammensetzung zur Behandlung oder Vorbeugung einer S.-aureus-Infektion, umfassend eine wirksame Menge eines Antikörpers nach Anspruch 1 und ein pharmazeutisch annehmbares Vehikel, einen pharmazeutisch annehmbaren Träger oder einen pharmazeutisch annehmbaren Exzipienten.

19. Verwendung eines Antikörpers nach Anspruch 1 zur Herstellung eines Medikaments zur Behandlung oder Vorbeugung einer S.-aureus-Infektion in einem menschlichen oder tierischen Patienten.

20. Verwendung eines isolierten S.-aureus-Map10-Proteins, das eine Aminosäuresequenz gemäß Seq.-ID Nr. 2 aufweist, zur Herstellung eines Medikaments zur Behandlung oder Vorbeugung einer S.-aureus-Infektion.

21. In-vitro-Verfahren zur Identifikation von Antikörpern gegen das Map10-Protein, umfassend das Zusetzen eines isolierten Map10-Proteins, das eine Aminosäuresequenz gemäß Seq.-ID Nr. 2 aufweist, zu einer Probe, die vermutlich Anti-MAP-Antikörper enthält, und das Bestimmen, ob Antikörper an das zugesetzte Map10-Protein gebunden haben.

22. Isolierter Antikörper nach Anspruch 1, der die Fähigkeit aufweist, an eine Aminosäuresequenz zu binden, für welche die Nucleinsäuresequenz der Seq.-ID Nr. 1 oder eine degenerierte Form davon kodiert.

23. Isoliertes S.-aureus-Map10-Protein, das die Aminosäuresequenz gemäß Seq.-ID Nr. 2 aufweist.

24. Isoliertes Protein nach Anspruch 23, das eine Aminosäuresequenz aufweist, für welche eine Nucleinsäuresequenz gemäß Seq.-ID Nr. 1 oder eine degenerierte Form davon kodiert.

25. Isolierte Nucleinsäuresequenz, die aus einer Nucleinsäuresequenz gemäß Seq.-ID Nr. 1 besteht.

26. Antikörper nach Anspruch 1 zur Verwendung bei der Behandlung oder Vorbeugung einer S.-aureus-Infektion in einem menschlichen oder tierischen Patienten.

27. Isoliertes S.-aureus-Map10-Protein, das eine Aminosäuresequenz gemäß Seq.-ID Nr. 2 aufweist, zur Behandlung oder Vorbeugung einer S.-aureus-Infektion.

## Revendications

1. Anticorps isolé qui se lie à la protéine Map10 de S. aureus, où la protéine Map10 a une séquence selon SEQ ID NO:2.

2. Anticorps selon la revendication 1, où ledit anticorps empêche une infection par S. aureus chez un être humain ou un animal.

3. Anticorps selon la revendication 1, où ledit anticorps inhibe la liaison des bactéries staphylococciques à des cellules eucaryotes.

4. Anticorps selon la revendication 1, où ledit anticorps est approprié pour une administration parentérale, orale, intranasale, sous-cutannée, en aérosol ou intraveineuse à un humain ou un animal.

5. Anticorps selon la revendication 1, où l'anticorps est un anticorps monoclonal.

6. Anticorps selon la revendication 5, où l'anticorps monoclonal est d'un type sélectionné dans le groupe consistant en anticorps chimériques, humanisés et monoclonaux humains.

7. Anticorps selon la revendication 5, où l'anticorps est un anticorps monoclonal monocaténaire.

8. Anticorps selon la revendication 1 qui comprend un fragment d'anticorps ayant la même spécificité de liaison d'un anticorps qui se lie à la protéine MAP de S. aureus.

9. Anticorps selon la revendication 1 ayant une séquence légère variable en accord avec SEQ ID NO:4.

10. Anticorps selon la revendication 1 ayant une séquence légère variable codé par une séquence d'acide nucléique en accord avec SEQ ID NO:3 ou ses dégénérés.

11. Anticorps selon la revendication 1 ayant une séquence lourde variable en accord avec SEQ ID NO:6.

12. Anticorps selon la revendication 1 ayant une séquence lourde variable codée par une séquence d'acide nucléique selon SEQ ID NO:1 ou ses dégénérés.

13. Anticorps selon la revendication 1 où l'anticorps est un anticorps polyclonal.

14. Antisérum isolé contenant un anticorps selon la revendication 1.

15. Kit de diagnostic comprenant un anticorps selon la revendication 1 et des moyens pour détecter la liaison par cet anticorps.

16. Kit de diagnostic selon la revendication 15 où ledit moyen pour détecter la liaison comprenant un marqueur détectable qui est enchaîné audit anticorps.

17. Méthode in vivo pour diagnostiquer une infection par S. aureus consistant à ajouter un anticorps selon la revendication 1 à un échantillon suspecté d'être infecté par S. aureus et à déterminer si des anticorps se sont liés à l'échantillon.

18. Composition pharmaceutique pour le traitement ou la prévention d'une infection par S. aureus comprenant une quantité efficace de l'anticorps de la revendication 1 et un véhicule, support ou récipient pharmaceutiquement acceptable.

19. Utilisation d'un anticorps selon la revendication 1 dans la préparation d'un médicament pour le traitement ou la prévention d'une infection par S. aureus chez un patient humain ou animal.

20. Utilisation d'une protéine Map10 de S. aureus isolée ayant une séquence d'acides aminés selon SEQ ID NO:2 dans la préparation d'un médicament pour le traitement ou la prévention d'une infection par S. *aureus*.

21. Méthode *in vitro* d'identification d'anticorps à la protéine Map10 comprenant l'addition d'une protéine Map10 isolée ayant une séquence d'acides aminés selon SEQ ID NO:2 à un échantillon suspecté de contenir des anticorps anti-MAP et la détermination du fait que des anticorps se sont liés à la protéine MAP ajoutée.

22. Anticorps isolé selon la revendication 1 qui a l'aptitude à se lier à une séquence d'acides aminés codée par la séquence d'acide nucléique, de SEQ ID NO:1 ou ses dégénérés.

23. Protéine Map10 de S. Aureus isolée ayant une séquence d'acides aminés selon SEQ ID NO:2.

24. Protéine isolée selon la revendication 23 ayant une séquence d'acides aminés codée par une séquence d'acide nucléique selon SEQ ID NO:1 ou ses dégénérés.

25. Séquence d'acide nucléique isolé consistant en une séquence d'acide nucléique selon SEQ ID NO:1.

26. Anticorps selon la revendication 1 à utiliser dans le traitement ou la prévention d'une infection par S. aureus chez un patient humain ou animal.

27. Protéine Map10 de S. aureus isolée ayant une séquence d'acides aminés selon SEQ ID NO:2 pour le traitement ou la prévention d'une infection par *S. aureus.*
